# EUROPÄISCHE PATENTSCHRIFT

(11) **EP 3 273 887 B1**
(45) Veröffentlichungstag und Bekanntmachung des Hinweises auf die Patenterteilung: **01.05.2019**
(21) Anmeldenummer: 16720724.0
(22) Anmeldetag: 21.03.2016
(51) Int. Cl.: A61B 17/70

(54) **INSTRUMENT ZUM VERBINDEN EINES KORREKTURSTABS MIT EINER KNOCHENSCHRAUBE**
INSTRUMENT FOR CONNECTING A CORRECTION ROD TO A BONE SCREW
INSTRUMENT PERMETTANT DE RELIER UNE TIGE CORRECTRICE À UNE VIS OSSEUSE

(30) Priorität: 24.03.2015 DE 102015205362
(43) Veröffentlichungstag der Anmeldung: 31.01.2018
(73) Patentinhaber: Silony Medical International AG, 8500 Frauenfeld (CH)
(72) Erfinder: HEUER, Frank, 70794 Filderstadt (DE)
(74) Vertreter: DREISS Patentanwälte PartG mbB
(86) Internationale Anmeldenummer: PCT/EP2016/056167
(87) Internationale Veröffentlichungsnummer: WO 2016/150920

(56) Entgegenhaltungen:
- DE-A1-102013 207 183
- US-A1- 2005 149 053
- US-A1- 2008 234 765
- US-A1- 2010 185 248
- US-A1- 2013 066 385

## Beschreibung

Die Erfindung betrifft ein Instrument zum Verbinden eines Korrekturstabs mit einer Knochenschraube, insbesondere einer Pedikelschraube in der Wirbelsäulenchirurgie, mit einer axialen Längsrichtung, mit einem außenliegenden ersten Gehäuseteil, welches einen zylindrischen ein Innengewinde aufweisenden hohlen Abschnitt und einen ungefähr halbschalenförmigen Abschnitt aufweist, der einen ersten Klammerschenkel bildet, mit einem ungefähr halbschalenförmigen außenliegenden zweiten Gehäuseteil, welches einen zweiten Klammerschenkel bildet, der an dem ersten Klammerschenkel um eine orthogonal zu der axialen Längsrichtung verlaufende Schwenkachse begrenzt schwenkbar angelenkt ist, und mit einem die außenliegenden Gehäuseteile axial durchsetzenden und damit überwiegend innenliegenden Einstellteil, welches an einem Abschnitt ein Außengewinde aufweist und damit in das Innengewinde des außenliegenden ersten Gehäuseteils einschraubbar ist, wobei ein proximales Ende des Einstellteils hierfür von außerhalb der Gehäuseteile manuell ergreifbar ist, und mit einem Stabdruckteil, welches gegen den Korrekturstab axial anlegbar ist und mit dem Einstellteil axial gekoppelt ist jedoch gegenüber dem Einstellteil um die axiale Längsrichtung drehbar ist, so dass Drehbewegungen des Einstellteils nicht auf das Stabdruckteil übertragen werden, und wobei die Klammerschenkel das Stabdruckteil schalenförmig umgeben und längsverschieblich führen und jeweils ein distales Ende und ein proximales Ende aufweisen, wobei sie mit ihrem jeweiligen distalen Ende an der Knochenschraube angreifen und an ihrem proximalen Ende vom benutzenden Chirurgen durch Fingerdruck betätigbar sind.

Ein derartiges Instrument ist bekannt aus DE 10 2013 207 183 A1 der Anmelderin. Bei einem solchen vorbekannten Instrument liegen der erste und der zweite Klammerschenkel beim Ergreifen einer Pedikelschraube jeweils gegen einen Schenkel eines von der Seite betrachtet U-förmigen Aufnahmeteils der Pedikelschraube an, so dass die jeweilige Schalenform der Klammerschenkel ungefähr konzentrisch zu den Schenkeln des U-förmigen Aufnahmeteils verlaufen und zwischen sich den durch die U-Form gebildeten Aufnahmeraum für einen Korrekturstab freigeben. Somit ist also der Korrekturstab bzw. dessen Längserstreckung im Wesentlichen parallel zur Schwenkachse des Gelenks der beiden Klammerschenkel des Instruments. Dieses Klammergelenk benötigt jedoch verhältnismäßig großen Bauraum in Richtung der Schwenkachse. Hierdurch wird aber der Platz neben der betrachteten Pedikelschraube in Richtung der Längsachse des Korrekturstabs begrenzt, so dass eine benachbarte Pedikelschraube nicht ohne weiteres gleichzeitig mit einem weiteren Instrument ergriffen werden kann.

Der vorliegenden Erfindung liegt die Aufgabe zugrunde, ein Instrument der genannten Art im Hinblick auf seine praktische Einsetzbarkeit zu verbessern.

Diese Aufgabe wird bei einem Instrument der genannten Art erfindungsgemäß dadurch gelöst, dass die distalen Enden der beiden Klammerschenkel so ausgebildet sind, dass sie eine Aufnahmeöffnung mit einer radialen Querrichtung für die Aufnahme des Korrekturstabs in dieser radialen Querrichtung aufweisen und diese radiale Querrichtung orthogonal zu der Schwenkachse der beiden Klammerschenkel verläuft. Auf diese Weise wird erfindungsgemäß erreicht, dass beim Einsatz des Instruments während des chirurgischen Eingriffs die Anordnung bzw. Ausrichtung des Instruments und eines typischerweise U-förmigen Aufnahmeteils der Pedikelschraube derart ist, dass die Längserstreckung des Korrekturstabs oder bei etwaiger Biegung des Korrekturstabs seine tangentiale Erstreckung im Bereich des Aufnahmeteils der Pedikelschraube ungefähr orthogonal zur Schwenkachse der beiden Klammerschenkel des Instruments verläuft. Der ausladende Bereich des Schwenkgelenks der Klammerschenkel steht somit quer zur Längsrichtung oder Längserstreckung des Korrekturstabs seitlich vor, so dass auf diese Weise in Längsrichtung des Korrekturstabs neben der betreffenden Pedikelschraube Platz eingespart wird. Es kann somit mit einem weiteren entsprechenden Instrument eine sehr nahe benachbarte weitere Pedikelschraube ergriffen und mit dem Korrekturstab fixiert werden. Die Erfindung erweist sich als besonders vorteilhaft, da entlang der Stabrichtung mehr Platz zur Verfügung steht als bei vorbekannten Instrumenten. Es können gleichzeitig mehrere Instrumente entlang des Stabs gleichzeitig zum Fixieren von Korrekturstab und betreffender Pedikelschraube eingesetzt werden. Die erfindungsgemäße Lösung impliziert weiter, dass jeder Schenkel des U-förmigen Aufnahmeteils der Pedikelschraube von jeweils beiden Klammerschenkeln des Instruments ergriffen wird. Auch auf diese Weise ließe sich der grundlegende Erfindungsgedanke alternativ oder zusätzlich beschreiben.

Die erfindungsgemäße Lösung könnte durch geeignete Aufsätze oder Ansätze bei den beiden Klammerschenkeln ausgebildet sein. Es erweist sich nach einer Ausführungsform der Erfindung indessen als vorteilhaft, wenn die distalen Enden der beiden Klammerschenkel jeweils einen in der axialen Längsrichtung erstreckten und in radialer Richtung durchgehenden Schlitz aufweisen, welcher an der distalen Stirnseite der distalen Enden frei ausmündet, so dass der Korrekturstab orthogonal zu seiner Längserstreckung und in der axialen Längsrichtung des Instruments in diesen Schlitz einführbar ist. Jeder Klammerschenkel umfasst also zwei Stege, die zwischen sich den zumindest ungefähr in der radialen Querrichtung erstreckten Korrekturstab aufnehmen.

Nach einer Ausführungsform der Erfindung weist jeder Klammerschenkel zwei in axialer Längsrichtung erstreckte Klammerfinger auf, die in konzentrisch zur axialen Längsrichtung verlaufender Umfangsrichtung voneinander beabstandet sind. Die betreffenden Klammerfinger sind vorzugsweise einstückige Teile oder Bereiche der Klammerschenkel. Die Beabstandung ist wenigstens so groß ist wie der Durchmesser des Korrekturstabs und erstreckt sich auch in axialer Längsrichtung, damit der Korrekturstab darin verschieblich aufgenommen werden kann.

In Weiterbildung der Erfindung erweist es sich als vorteilhaft, wenn die beiden Klammerfinger eines Klammerschenkels in einem distalen Endbereich und im Bereich ihrer Beabstandung in Umfangsrichtung aufeinander zugewandte Vorsprünge aufweisen, welche gegen verschiedene Schenkel eines U-förmigen Aufnahmeteils der Knochenschraube für den Korrekturstab anlegbar sind und dabei eine Verdrehsicherung zwischen Knochenschraube und Instrument bilden.

Weiter erweist es sich als vorteilhaft, wenn die Klammerschenkel in einem distalen Endbereich radial innen konzentrisch erstreckte Stege aufweisen, die in konzentrisch erstreckte Nuten an der Außenseite der Schenkel eines U-förmigen Aufnahmeteils der Knochenschraube eingreifen können, um eine lösbare formschlüssige Kopplung in axialer Längsrichtung zwischen Knochenschraube und Instrument zu bilden.

Weiter erweist es sich als vorteilhaft, wenn das Stabdruckteil bei zunehmender axialer Zustellbewegung des Stabdruckteils in Richtung auf die Knochenschraube bzw. den Korrekturstab eine formschlüssige Kopplung mit wenigstens einem Klammerschenkel eingeht, so dass eine Öffnungsbewegung des betreffenden Klammerschenkels durch Verschwenkung um die Schwenkachse verhindert ist. Auf diese Weise wird sichergestellt, dass sich bei größtem axialen Druck, wenn also vermittels des Stabdruckteils der Korrekturstab und die Pedikelschraube in axialer Richtung gegeneinander gedrückt werden, das Instrument von dem U-förmigen Aufnahmeteil der Pedikelschraube nicht lösen kann.

In Weiterbildung dieses Erfindungsgedankens wird vorgeschlagen, dass das Stabdruckteil mit einem radial vorstehenden Ansatz zwischen den Klammerfingern wenigstens eines Klammerschenkels nach radial außen hindurchgreift und bei zunehmender axialer Zustellbewegung des Stabdruckteils in Richtung auf die Knochenschraube bzw. den Korrekturstab die Klammerfinger umgreift und so eine Öffnungsbewegung des betreffenden Klammerschenkels durch Verschwenkung um die Schwenkachse verhindert. Hierfür kann es sich als vorteilhaft erweisen, wenn der radial vorstehende Ansatz des Stabdruckteils in einer Schnittebene orthogonal zu der axialen Längsrichtung betrachtet T-förmig oder pilzförmig ausgebildet ist. Auf diese Weise kann sich der radial vorstehende Ansatz mit den seitlich abstehenden Bereichen der T- oder Pilzform von radial außerhalb über die Klammerfinger schieben und so eine Verschwenkung der Klammerschenkel um die Schwenkachse verhindern.

Nach einem weiteren Ausführungsbeispiel wird vorgeschlagen, dass bei einem Schwenkgelenk für die beiden Klammerschenkel wenigstens ein Gelenkstift nach radial innen vorsteht und mit seinem inneren Ende in eine in axialer Längsrichtung erstreckte Führungsnut an der radialen Außenseite des Stabdruckteils eingreift und dieses so gegen Verdrehen sichert jedoch in Längsrichtung längsverschieblich führt. Auf diese Weise wird erreicht, dass das Stabdruckteil einerseits gegenüber den halbschalenförmigen Gehäuseteilen unverdrehbar gestellt ist, zugleich aber unabhängig von der Schwenkstellung der Klammerschenkel in der Längsrichtung bezüglich des außen liegenden ersten Gehäuseteils des Instruments definiert ausgerichtet und geführt ist. Diese Ausbildung kann auf beiden Seiten des Gelenks so verwirklicht werden (nicht beansprucht).

In weiterer Ausbildung dieses Erfindungsgedankens erweist es sich als vorteilhaft, wenn die in axialer Längsrichtung erstreckte Führungsnut an der radialen Außenseite des Stabdruckteils in Richtung auf das distale Ende der Klammerschenkel V-förmig sich erweiternd ausmündet. Auf diese Weise findet eine selbständige Zentrierung beim Einführen des Stabdruckteils in distaler Richtung entlang des außen liegenden ersten Gehäuseteils statt.

Weiterhin wird beschrieben aber nicht Schutz in Anspruch genommen, ein Instrument mit den Merkmalen des Oberbegriffs des Anspruchs 1, wobei wenigstens ein Klammerschenkel zwei in axialer Längsrichtung erstreckte Klammerfinger aufweist, die in konzentrisch zur axialen Längsrichtung verlaufender Umfangsrichtung voneinander beabstandet sind, und wobei das Stabdruckteil mit einem radial vorstehenden Ansatz zwischen den Klammerfingern des Klammerschenkels nach radial außen hindurchgreift und bei zunehmender axialer Zustellbewegung des Stabdruckteils in Richtung auf die Knochenschraube bzw. den Korrekturstab die Klammerfinger umgreift und so eine Öffnungsbewegung des betreffenden Klammerschenkels durch Verschwenkung um die Schwenkachse verhindert. Vorzugsweise ist diese Ausbildung bei beiden Klammerschenkeln oder Klammerschalen so verwirklicht.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Instruments. In der Zeichnung zeigt:
Figur 1 eine explosionsartige Ansicht des erfindungsgemäßen Instruments;
Figur 2 eine perspektivische Ansicht der außenliegenden Gehäuseteile und des darin ein schraubbaren Einstellteils und des Stabdruckteils;
Figur 3 eine perspektivische Ansicht des Instruments nach Figur 1, wobei Klammerschenkel des Instruments an einer Knochenschraube festgelegt sind und ein Korrekturstab in dem U-förmigen Aufnahmeteil der Knochenschraube eingelegt ist, mit noch nicht anliegendem Stabdruckteil;
Figur 4 eine perspektivische Ansicht des Instruments nach Figur 3 mit gegen den Korrekturstab anliegenden Stabdruckteil;
Figur 5 eine perspektivische Ansicht des distalen Endes des Instruments und
Figur 6 eine perspektivische Ansicht entsprechend Figur 5 mit einer Schnittebene VI-VI in Figur 5.

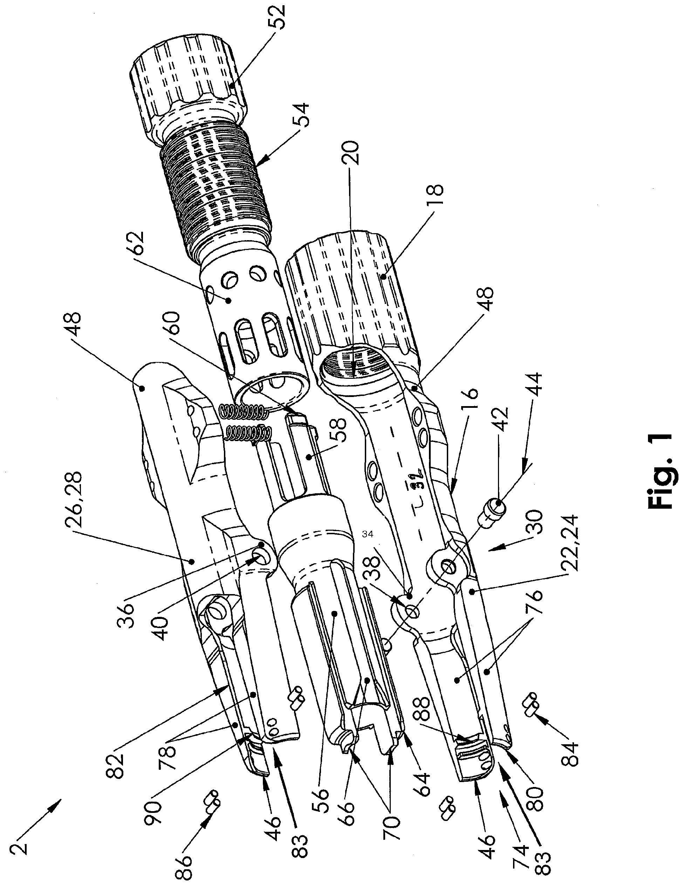

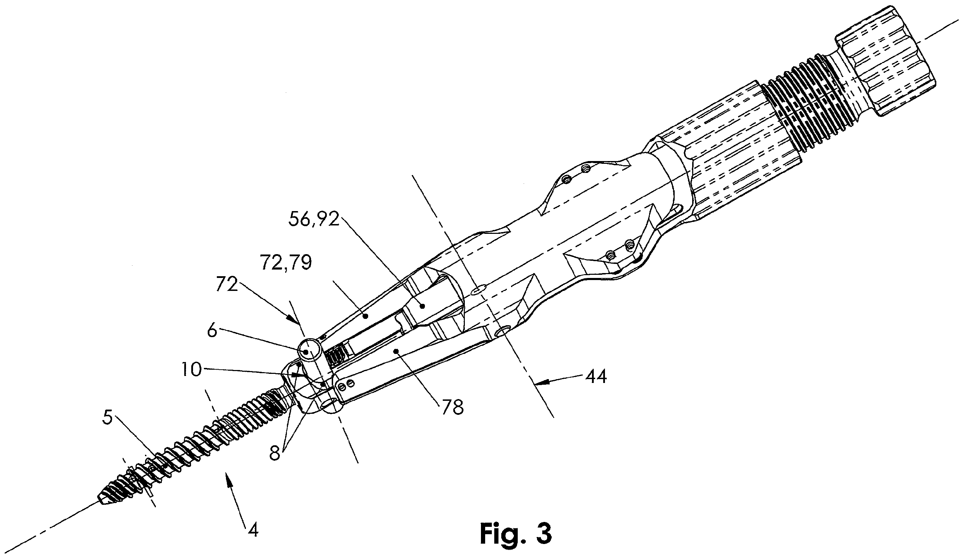

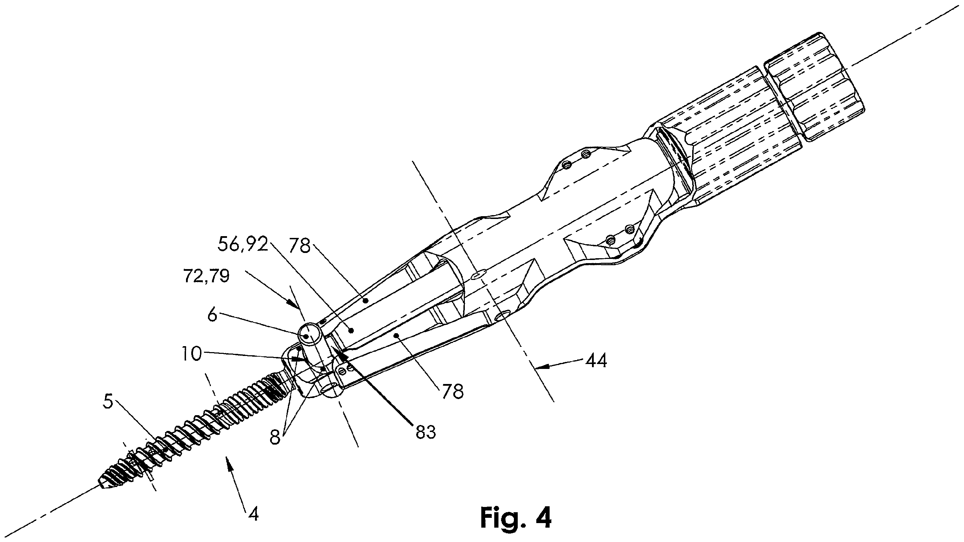

Figur 1 zeigt in explosionsartiger und perspektivischer Darstellung die wesentlichen Komponenten eines insgesamt mit dem Bezugszeichen 2 bezeichneten Instruments, welches auch häufig als Repositionsinstrument bezeichnet wird. Es dient einerseits zum Ergreifen und Halten einer Knochenschraube 4 mit Gewindeschaft 5, insbesondere einer Pedikelschraube, und zum korrekten Positionieren eines Korrekturstabs 6 an einer bereits in den Knochen eingeschraubten Knochenschraube 4. Im Zuge dieses Positionierens wird die Knochenschraube 4 wie in Figuren 3 und 4 dargestellt in noch näher zu beschreibender Weise von radial außen an ihren beiden Schenkeln 8 eines U-förmigen Aufnahmeteils 10 für den Korrekturstab ergriffen, wobei auf ebenfalls noch zu beschreibende Weise der Korrekturstab 6 gegen das U-förmige Aufnahmeteil 10 gedrückt und in einer bestimmungsgemäßen Position durch ein Schraubenmittel, insbesondere eine Madenschraube, fixiert wird. Dies geschieht in ebenfalls bekannter Weise, indem das Schraubenmittel mittels eines geeigneten Instruments in Längsrichtung durch das innen hohl ausgebildete Instrument 2 hindurchgeführt und in das U-förmige Aufnahmeteil 10 der Knochenschraube eingeschraubt wird. Das Instrument 2 kann auch vor dem Einlegen des Korrekturstabs 6 zum Ergreifen und Halten der Knochenschraube 4 und zum Einschrauben des Gewindeschafts 5 in den Knochen benutzt werden, indem auch hierfür durch das hohle Instrument 2 hindurch mit einem Werkzeug Zugriff auf eine Werkzeugansetzstelle am proximalen Ende des Gewindeschafts 5 genommen wird.

Das Instrument 2 umfasst ein außen liegendes erstes Gehäuseteil 16, welches einen zylindrischen, innen hohlen Abschnitt 18 mit einem Innengewinde 20 und einen halbschalenförmigen Abschnitt 22 aufweist. Dieser halbschalenförmige Abschnitt 22 bildet einen ersten Klammerschenkel 24 des Instruments. Des Weiteren umfasst das Instrument ein ungefähr halbschalenförmiges außen liegendes zweites Gehäuseteil 26, welches einen zweiten Klammerschenkel 28 bildet und hierfür über ein Schwenkgelenk 30 mit dem ersten Klammerschenkel 24 beschränkt schwenkbar verbunden ist. Hierfür weisen der erste und zweite Klammerschenkel 24 und 28 jeweils zwei aufeinander zu erstreckte und gegeneinander anlegbare Gelenklaschen 34, 36 mit einander fluchtenden Öffnungen 38, 40 auf, durch die sich beidseits ein Gewindestift 42 hineinerstreckt und hierdurch eine Schwenkachse 44 der beiden Klammerschenkel 24, 28 definiert. Man unterscheidet außerdem bei den Klammerschenkeln 24, 28 ein jeweiliges distales Ende 46 oder einen distalen Endbereich und ein proximales Ende 48 oder einen proximalen Endbereich. Zwischen den proximalen Endbereichen der Klammerschenkel 24, 28 sind Druckfedermittel derart angeordnet, dass sie die Klammerschenkel 24, 28 um die Schwenkachse 44 derart vorspannen, dass ihre distalen Enden 46 gegeneinander gezwungen werden, um durch formschlüssigen Eingriff eine Knochenschraube 4 ergreifen und halten zu können. Der zweite Klammerschenkel 28 komplettiert gewissermaßen das ungefähr hohlzylindrisch anmutende außenliegende Gehäuse des Instruments 2.

Des Weiteren umfasst das Instrument 2 ein die bislang beschriebenen Gehäuseteile 16, 26 axial durchsetzendes innen liegendes Einstellteil 52, welches mit einem Außengewinde 54 in das Innengewinde 20 des zylindrischen Abschnitts 18 des ersten Gehäuseteils 16 einschraubbar ist, sowie ein Stabdruckteil 56, welches mit dem Einstellteil 52 in axialer Längsrichtung 32 gekoppelt, jedoch gegenüber dem Einstellteil 52 um die axiale Längsrichtung 32 drehbar ist. Hierfür weist das Stabdruckteil 56 an seinem proximalen Ende ungefähr in axialer Längsrichtung 32 und in Richtung auf das Einstellteil 52 erstreckte quer zur Längsrichtung 32 etwas nachgiebige Raststege 58 auf, die jeweils einen in radialer Richtung erstreckten Vorsprung 60 aufweisen. Das Einstellteil 52 weist einen hiermit zusammenwirkenden Aufnahmeabschnitt 62 mit einer korrespondierenden nicht dargestellten Umfangsnut auf, in welche die Vorsprünge 60 der Raststege 58 beim axialen Fügen der beiden Teile einrasten können. Auf diese Weise besteht eine Kopplung der Teile in axialer Längsrichtung 32 und eine Verdrehbarkeit des Stabdruckteils 56 gegenüber dem Einstellteil 52.

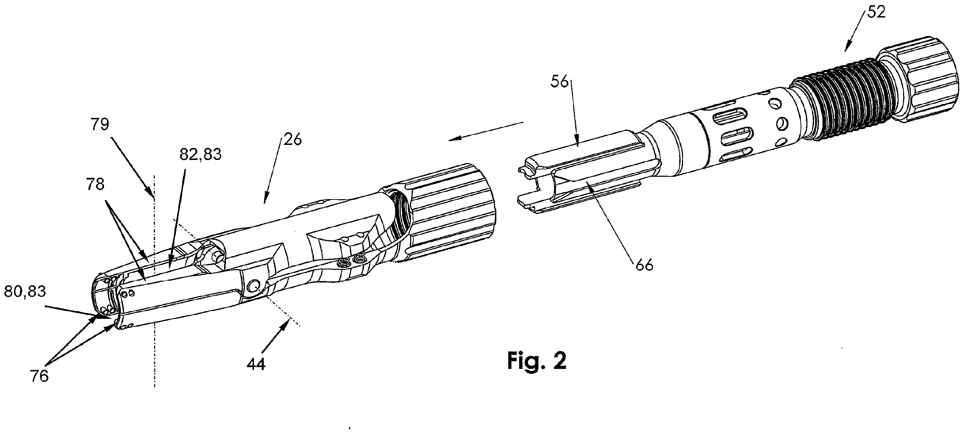

Das Stabdruckteil 56 ist ebenfalls ungefähr hohlzylindrisch ausgebildet. Es weist an seinem Außenumfang 64 auf diametral gegenüberliegenden Seiten je eine in axialer Längsrichtung 32 erstreckte Führungsnut 66 auf, in welche die beiden Gelenkstifte 42 mit ihrem freien Ende eingreifen. Auf diese Weise ist das Stabdruckteil 56 längsverschieblich, jedoch unverdrehbar in den beiden außen liegenden Gehäuseteilen 16, 26 aufgenommen. Die Führungsnut 66 ist am distalen Ende V-förmig erweitert. Hierdurch wird eine Zentrierhilfe beim Einschieben des Stabdruckteils 56 und des damit gekoppelten Einstellteils 52 in axialer Längsrichtung 32 in das Gehäuse realisiert (s. Figur 2). Durch die sich V-förmig erweiternden Führungsnuten 66 gelangt das Stabdruckteil 56 ohne weiteres in seine korrekte Position, in der die Gewindestifte 42 in die Führungsnuten 66 eingreifen und hierdurch die korrekte Betriebsposition des Stabdruckteils relativ zu den Gehäuseteilen 16, 26 vorgeben.

Die weitere Verlagerung des Stabdruckteils 56 in distaler Richtung erfolgt durch Einschrauben des Einstellteils 52 in das Innengewinde 20 des außen liegenden ersten Gehäuseteils 16. Mit zunehmender Verlagerung des Stabdruckteils 56 in distaler Richtung gelangen schließlich radial gegenüberliegende Stirnwangen 70 am distalen Ende des Stabdruckteils 56 in axiale Anlage gegen den Korrekturstab 6, während die Klammerschenkel 24, 28 auf noch zu beschreibende Weise die Pedikelschraube fixiert halten. Durch weiteres Einschrauben des Einstellteils 52 drückt das Stabdruckteil den Stab weiter in Richtung auf den Grund des U-förmigen Aufnahmeteils 10 der Knochenschraube. Schließlich wird wie eingangs erwähnt der Korrekturstab 6 mittels eines Schraubenmittels gegen das Aufnahmeteil 10 festgelegt.

Die außen liegenden Gehäuseteile 16 und 26 bzw. die von ihnen gebildeten Klammerschenkel 24, 28 und das Stabdruckteil 56 sind erfindungsgemäß so ausgebildet, dass die Knochenschraube 4 bzw. deren U-förmiges Aufnahmeteil 10 für den Korrekturstab 6 so ergriffen und in der ergriffenen Position fixiert werden kann, dass eine Längserstreckung oder Orientierung 72 des Korrekturstabs 6 orthogonal zu der Schwenkachse 44 der beiden Klammerschenkel 24, 28 verläuft. Auf diese Weise ist das in Richtung der Schwenkachse 44 verhältnismäßig breit bauende Schwenkgelenk 30 nicht in Stablängsrichtung, sondern quer dazu angeordnet. Dies schafft Platzersparnis in Richtung der Längserstreckung 72 des Korrekturstabs 6. In der Folge können auf engerem Raum mehrere Instrumente gleichzeitig zum Ergreifen von nahe beieinander eingeschraubten Knochenschrauben eingesetzt werden.

Die beiden ungefähr halbschalenförmigen Klammerschenkel 24, 28 sind im Bereich distal zu der Schwenkachse 44 in konzentrisch zur axialen Längsrichtung 32 erstreckter Umfangsrichtung 74 nicht durchgehend, sondern sie umfassen jeweils zwei in axialer Längsrichtung 32 erstreckte Klammerfinger 76 bzw. 78. Diese Klammerfinger 76 einerseits und 78 andererseits sind voneinander in Umfangsrichtung 74 beabstandet. Zwischen ihnen ist ein in axialer Längsrichtung 32 erstreckter und in radialer Querrichtung 79 durchgehender Schlitz 80 bzw. 82 ausgebildet, der eine Aufnahmeöffnung 83 für den Korrekturstab 6 bildet. Die Breite dieses Schlitzes 80, 82 in Umfangsrichtung 74 ist zumindest geringfügig größer als der Durchmesser des Korrekturstabs 6, so dass er diesen aufzunehmen vermag, und zwar auch in geringfügig gebogenem Zustand. Der Korrekturstab 6 ist dann mit seiner Längserstreckung 72 exakt oder aufgrund geringfügiger Biegung ungefähr in der radialen Querrichtung 79 der Aufnahmeöffnung 83 erstreckt, die erfindungsgemäß orthogonal zur Schwenkachse 44 der Klammerschenkel 24, 28 verläuft.

Weiter umfassen die Klammerfinger 76 bzw. 78 im Bereich des Schlitzes 80, 82 einander zugewandte, insbesondere stiftförmige Vorsprünge 84, 86, welche das U-förmige Aufnahmeteil 10 der Knochenschraube 6 derart umgreifen, dass sie die Schenkel 8 des U-förmigen Aufnahmeteils 10 in Umfangsrichtung 74 lagefixieren, so dass eine Verdrehung des U-förmigen Halteteils 10 gegenüber dem Instrument 2 nicht möglich ist. Die axiale Kopplung bzw. Fixierung wird mittels konzentrischer Ringstege 88, 90 an der radial inneren Seite der Klammerfinger 76 bzw. 78 realisiert, welche in konzentrische Nuten am Außenumfang der Schenkel 8 des U-förmigen Halteteils 10 eingreifen. Beim Ergreifen der Knochenschraube 4 wird also jeder Schenkel 8 des U-förmigen Aufnahmeteils 10 von beiden Klammerschenkeln 24, 28 auf gegenüberliegenden Seiten kontaktiert bzw. gegriffen. Daher ist sich die Längserstreckung 72 des Stabs 6 orthogonal zur Schwenkachse 44.

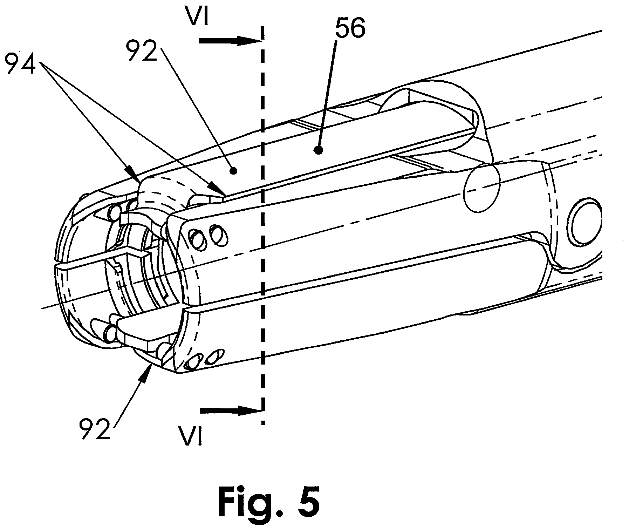

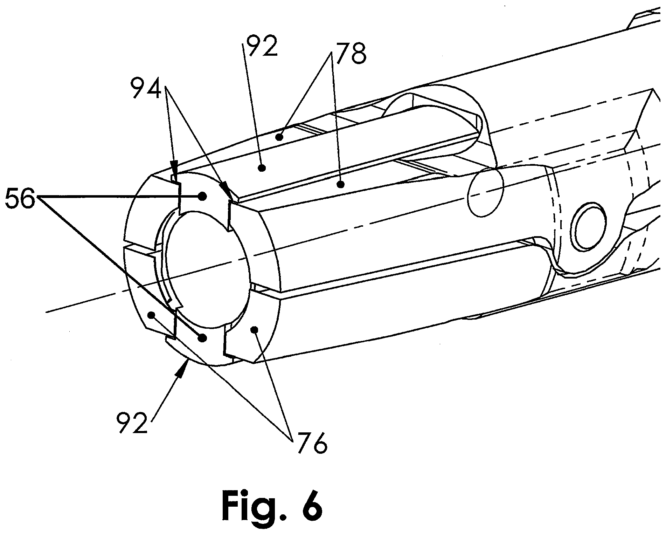

Die Figuren 5 und 6 zeigen eine weitere Besonderheit des erfindungsgemäßen Instruments. Das Stabdruckteil 56 ist derart ausgebildet und wirkt mit vorzugsweise beiden Klammerschenkeln 24, 28 derart zusammen, dass eine Öffnungsbewegung der Klammerschenkel durch Verschwenkung um die Schwenkachse 44 verhindert ist, wenn das Stabdruckteil 56 eine hinreichende Stellbewegung in Richtung auf das distale Ende 46 der Klammerschenkel oder des Instruments ausgeführt hat. Hierfür umfasst das Stabdruckteil 56 auf diametral gegenüberliegenden Seiten je einen radial vorstehenden Ansatz 92. Mit diesem radialen Ansatz 92 greift das Stabdruckteil 56 durch den Schlitz 80, 82 zwischen den jeweiligen Klammerfingern 76 bzw. 78 nach außen hindurch. Der Ansatz 92 ist dabei in der Schnittebene der Figur 6 betrachtet T- oder pilzförmig ausgebildet. Er vermag dann mit den quer verlaufenden Schenkeln 94 der T- oder Pilzform die Außenseite der Klammerfinger 76 bzw. 78 zu übergreifen, und zwar in dem Bereich distal zur Schwenkachse 44, so dass sich die Klammerfinger 76, 78 bzw. Klammerschenkel 24, 28 nicht mehr distal zu öffnen vermögen. Auf diese Weise wird verhindert, dass sich beim Einleiten von Kräften im Betrieb des Instruments die Klammerschenkel unbeabsichtigt aus ihrem Hintergriff an der Außenseite des U-förmigen Aufnahmeteils der Pedikelschraube lösen können. Im beispielhaft dargestellten Fall sind die jeweiligen Klammerfinger 76 bzw. 78 im Kontaktbereich mit dem radialen Ansatz 92 abgeflacht ausgebildet, so dass die quer verlaufenden Schenkel 94 der T- oder Pilzform und die Klammerfinger 76 bzw. 78 flächenhaft aneinander anliegen, so dass auch eine Verteilung und gleichmäßige Einleitung der Kräfte erreicht wird, so dass es nicht infolge von Kraftspitzen zu Materialverformungen kommt. Jedenfalls wird durch diese weitere Ausgestaltung erfindungsgemäß erreicht, dass zum Zeitpunkt der Krafteinleitung, wenn also die Knochenschraube bzw. deren U-förmiges Aufnahmeteil 10 und der Korrekturstab 6 gegeneinander gezogen werden, eine absolut sichere axiale Verankerung des Instruments mit der Knochenschraube sichergestellt wird. Erst wenn das Stabdruckteil 56 durch Ausschrauben des Einstellteils 52 die Klammerfinger 76, 78 freigibt, indem sie außer Eingriff mit dem radialen Ansatz 92 gelangen, kann das Instrument wieder von der Knochenschraube gelöst werden. Figur 3 zeigt ungefähr den Bereich, an dem der radiale Ansatz 92 des Stabdruckteils 52 die Klammerfinger 78 zu übergreifen beginnt bzw. bei der entgegengesetzten Rückbewegung freigibt.

## Patentansprüche

1. Instrument (2) zum Verbinden eines Korrekturstabs (6) mit einer Knochenschraube (4), insbesondere einer Pedikelschraube in der Wirbelsäulenchirurgie, mit einer axialen Längsrichtung (32), mit einem außenliegenden ersten Gehäuseteil (16), welches einen zylindrischen ein Innengewinde (20) aufweisenden hohlen Abschnitt (18) und einen ungefähr halbschalenförmigen Abschnitt (22) aufweist, der einen ersten Klammerschenkel (24) bildet, mit einem ungefähr halbschalenförmigen außenliegenden zweiten Gehäuseteil (26), welches einen zweiten Klammerschenkel (28) bildet, der an dem ersten Klammerschenkel (24) um eine orthogonal zu der axialen Längsrichtung (32) verlaufende Schwenkachse (44) begrenzt schwenkbar angelenkt ist, und mit einem die außenliegenden Gehäuseteile (16, 26) axial durchsetzenden und damit innenliegenden Einstellteil (52), welches an einem Abschnitt ein Außengewinde (54) aufweist und damit in das Innengewinde (20) des außenliegenden ersten Gehäuseteils (16) einschraubbar ist, wobei ein proximales Ende des Einstellteils (52) hierfür von außerhalb der Gehäuseteile (16, 26) manuell ergreifbar ist, und mit einem Stabdruckteil (56), welches gegen den Korrekturstab (6) axial anlegbar ist und mit dem Einstellteil (52) axial gekoppelt ist jedoch gegenüber dem Einstellteil (52) um die axiale Längsrichtung (32) drehbar ist, so dass Drehbewegungen des Einstellteils (52) nicht auf das Stabdruckteil (56) übertragen werden, und wobei die Klammerschenkel (24, 28) das Stabdruckteil (56) schalenförmig umgeben und längsverschieblich führen und jeweils ein distales Ende (46) und ein proximales Ende (48) aufweisen, wobei sie mit ihrem jeweiligen distalen Ende (46) an der Knochenschraube (4) angreifen und an ihrem proximalen Ende (48) vom benutzenden Chirurgen durch Fingerdruck betätigbar sind, **dadurch gekennzeichnet, dass** die distalen Enden (46) der beiden Klammerschenkel (24, 28) so ausgebildet sind, dass sie eine Aufnahmeöffnung (83) mit einer radialen Querrichtung (79) für die Aufnahme des Korrekturstabs (6) in dieser radialen Querrichtung (79) aufweisen und diese radiale Querrichtung (79) orthogonal zu der Schwenkachse (44) der Klammerschenkel (24, 28) verläuft.

2. Instrument nach Anspruch 1, **dadurch gekennzeichnet, dass** die distalen Enden (46) der beiden Klammerschenkel (24, 28) jeweils einen in der axialen Längsrichtung (32) erstreckten und in radialer Richtung durchgehenden Schlitz (80, 82) aufweisen, welcher an der distalen Stirnseite der distalen Enden (46) frei ausmündet, so dass der Korrekturstab (6) orthogonal zu seiner Längserstreckung (72) und in der axialen Längsrichtung (32) des Instruments in diesen Schlitz (80, 82) einführbar ist.

3. Instrument nach Anspruch 1 oder 2, **dadurch gekennzeichnet, dass** jeder Klammerschenkel (24, 28) zwei in axialer Längsrichtung (32) erstreckte Klammerfinger (76, 78) aufweist, die in konzentrisch zur axialen Längsrichtung (32) verlaufender Umfangsrichtung (74) voneinander beabstandet sind.

4. Instrument nach Anspruch 3, **dadurch gekennzeichnet, dass** die beiden Klammerfinger (76, 78) eines Klammerschenkels (24, 28)in einem distalen Endbereich (46) und im Bereich ihrer Beabstandung in Umfangsrichtung (74) aufeinander zugewandte Vorsprünge (84, 86) aufweisen, welche gegen verschiedene Schenkel (8) eines U-förmigen Aufnahmeteils (10) der Knochenschraube (4) für den Korrekturstab (6) anlegbar sind und dabei eine Verdrehsicherung zwischen Knochenschraube (4) und Instrument (2) bilden.

5. Instrument nach einem oder mehreren der vorstehenden Ansprüche, **dadurch gekennzeichnet, dass** die Klammerschenkel (24, 28) in einem distalen Endbereich (46) radial innen konzentrisch erstreckte Stege (88) aufweisen, die in konzentrisch erstreckte Nuten an der Außenseite der Schenkel (8) eines U-förmigen Aufnahmeteils (10) der Knochenschraube (4) eingreifen können, um eine lösbare formschlüssige Kopplung in axialer Längsrichtung (32) zwischen Knochenschraube (4) und Instrument (2) zu bilden.

6. Instrument nach einem oder mehreren der vorstehenden Ansprüche, **dadurch gekennzeichnet, dass** das Stabdruckteil (56) bei zunehmender axialer Zustellbewegung des Stabdruckteils (56) in Richtung auf die Knochenschraube (4) bzw. den Korrekturstab (6) eine formschlüssige Kopplung mit wenigstens einem Klammerschenkel (24, 28) eingeht, so dass eine Öffnungsbewegung des betreffenden Klammerschenkels (24, 26) durch Verschwenkung um die Schwenkachse (44) verhindert ist.

7. Instrument nach Anspruch 6, **dadurch gekennzeichnet, dass** das Stabdruckteil (56) mit einem radial vorstehenden Ansatz (92) zwischen den Klammerfingern (76, 78) wenigstens eines Klammerschenkels (24, 28) nach radial außen hindurchgreift und bei zunehmender axialer Zustellbewegung des Stabdruckteils (56) in Richtung auf die Knochenschraube (4) und den Korrekturstab (6) die Klammerfinger (76, 78) umgreift und so eine Öffnungsbewegung des betreffenden Klammerschenkels (24, 28) durch Verschwenkung um die Schwenkachse (44) verhindert.

8. Instrument nach Anspruch 7, **dadurch gekennzeichnet, dass** der radial vorstehende Ansatz (92) des Stabdruckteils (56) in einer Schnittebene orthogonal zu der axialen Längsrichtung (32) betrachtet T-förmig oder pilzförmig ausgebildet ist.

9. Instrument nach einem oder mehreren der vorstehenden Ansprüche, **dadurch gekennzeichnet, dass** bei einem Schwenkgelenk (30) für die beiden Klammerschenkel (24, 28) wenigstens ein Gelenkstift (42) nach radial innen vorsteht und mit seinem inneren Ende in eine in axialer Längsrichtung (32) erstreckte Führungsnut (66) an der radialen Außenseite des Stabdruckteils (56) eingreift und dieses so gegen Verdrehen sichert jedoch in Längsrichtung (32) längsverschieblich führt.

10. Instrument nach Anspruch 9, **dadurch gekennzeichnet, dass** die in axialer Längsrichtung (32) erstreckte Führungsnut (66) an der radialen Außenseite des Stabdruckteils (56) in Richtung auf das distale Ende (46) der Klammerschenkel (24, 28) V-förmig sich erweiternd ausmündet.

## Claims

1. Instrument (2) for connecting a correction rod (6) to a bone screw (4), in particular a pedicle screw in spinal surgery, said instrument having an axial longitudinal direction (32) and comprising an external first housing part (16), which comprises a cylindrical hollow portion (18) having an internal thread (20) and a portion (22) that is approximately the shape of a half-shell and that forms a first clamp leg (24), said instrument comprising an external second housing part (26), which is approximately the shape of a half-shell and forms a second clamp leg (28) that is hinged on the first clamp leg (24) so as to be pivotable in a limited manner about a pivot axis (44) that extends orthogonally to the axial longitudinal direction (32), and said instrument comprising an adjustment part (52), which axially penetrates the external housing parts (16, 26) and is thus internal, and which comprises an external thread (54) on a portion thereof and can thus be screwed into the internal thread (20) of the external first housing part (16), a proximal end of the adjustment part (52) being manually graspable from outside the housing parts (16, 26) for this purpose, and said instrument comprising a rod pressure part (56), which can be axially placed against the correction rod (6) and which is axially coupled to the adjustment part (52) but can rotate about the axial longitudinal direction (32) relative to the adjustment part (52), such that rotational movements of the adjustment part (52) are not transmitted to the rod pressure part (56), and the clamp legs (24, 28) surrounding the rod pressure part (56) in a shell-like manner and guiding same in a longitudinally slidable manner, said clamp legs each comprising a distal end (46) and a proximal end (48), the respective distal end (46) engaging on the bone screw (4) and the proximal end (48) being actuatable by finger pressure of the operating surgeon, **characterized in that** the distal ends (46) of the two clamp legs (24, 28) are designed such that they comprise an accommodation opening (83) having a radial transverse direction (79) for accommodating the correction rod (6) in said radial transverse direction (79), and **in that** said radial transverse direction (79) extends orthogonally to the pivot axis (44) of the clamp legs (24, 28).

2. Instrument according to claim 1, **characterized in that** each of the distal ends (46) of the two clamp legs (24, 28) comprises a slot (80, 82) that extends in the axial longitudinal direction (32), is continuous in the radial direction and opens out in unobstructed manner at the distal end face of the distal ends (46) such that the correction rod (6) can be inserted into said slot (80, 82) orthogonally to the longitudinal extension (72) of said rod and in the axial longitudinal direction (32) of the instrument.

3. Instrument according to claim 1 or claim 2, **characterized in that** each clamp leg (24, 28) comprises two clamp fingers (76, 78) that extend in the axial longitudinal direction (32) and are mutually spaced apart in the peripheral direction (74) extending concentrically to the axial longitudinal direction (32).

4. Instrument according to claim 3, **characterized in that** the two clamp fingers (76, 78) of a clamp leg (24, 28) comprise mutually facing projections (84, 86) in a distal end region (46) and in the region of the spacing of said clamp fingers in the peripheral direction (74), which projections can be placed against different legs (8) of a U-shaped accommodation part (10) of the bone screw (4) for the correction rod (6) and thereby form an anti-rotation means between the bone screw (4) and the instrument (2).

5. Instrument according to one or more of the preceding claims, **characterized in that** the clamp legs (24, 28) comprise ribs (88) in a distal end region (46) that extend radially inwardly concentrically and that can engage in concentrically extending grooves on the outer face of the legs (8) of a U-shaped accommodation part (10) of the bone screw (4) in order to form a releasable positive coupling in the axial longitudinal direction (32) between the bone screw (4) and the instrument (2).

6. Instrument according to one or more of the preceding claims, **characterized in that** the rod pressure part (56) enters into positive coupling with at least one clamp leg (24, 28) during increasing axial feed motion of the rod pressure part (56) towards the bone screw (4) or correction rod (6), such that an opening movement of the relevant clamp leg (24, 28) produced by pivoting about the pivot axis (44) is prevented.

7. Instrument according to claim 6, **characterized in that** the rod pressure part (56) reaches through radially outwards between the clamp fingers (76, 78) of at least one clamp leg (24, 28) by means of a radially projecting end piece (92) and engages around the clamp fingers (76, 78) during increasing axial feed motion of the rod pressure part (56) towards the bone screw (4) and correction rod (6), and thus prevents an opening movement of the relevant clamp leg (24, 28) produced by pivoting about the pivot axis (44).

8. Instrument according to claim 7, **characterized in that** the radially projecting end piece (92) of the rod pressure part (56) is T-shaped or mushroom-shaped when viewed in a sectional plane orthogonal to the axial longitudinal direction (32).

9. Instrument according to one or more of the preceding claims, **characterized in that**, in a pivot joint (30) for the two clamp legs (24, 28), at least one joint pin (42) projects radially inwards and the inner end thereof engages in a guide groove (66) that extends in the axial longitudinal direction (32) on the radial outer face of the rod pressure part (56) and thus secures same against rotation but guides same in a longitudinally slidable manner in the longitudinal direction (32).

10. Instrument according to claim 9, **characterized in that** the guide groove (66) that extends in the axial longitudinal direction (32) opens out at the radial outer face of the rod pressure part (56) so as to widen in the manner of a V towards the distal end (46) of the clamp legs (24, 28).

## Revendications

1. Instrument (2) destiné à relier une tige correctrice (6) à une vis à os (4), en particulier à une vis pédiculaire en chirurgie de la colonne vertébrale, avec une direction longitudinale axiale (32), avec une première partie de boîtier (16) située à l'extérieur qui présente une section creuse (18) cylindrique ayant un filetage intérieur (20) ainsi qu'une section à peu près en forme de demi-coque (22) qui forme une première branche de pince (24), avec une deuxième partie de boîtier (26) à peu près en forme de demi-coque et située à l'extérieur qui forme une deuxième branche de pince (28) qui est articulée sur ladite première branche de pince (24) de manière à pouvoir pivoter de manière limitée autour d'un axe de pivotement (44) s'étendant orthogonalement à la direction longitudinale axiale (32), et avec une partie de réglage (52) qui traverse axialement lesdites parties de boîtier (16, 26) situées à l'extérieur et est située donc à l'intérieur et qui présente un filetage extérieur (54) sur une section et peut donc être vissée dans le filetage intérieur (20) de la première partie de boîtier (16) située à l'extérieur, dans lequel, à cette fin, une extrémité proximale de la partie de réglage (52) peut être saisie manuellement depuis l'extérieur des parties de boîtier (16, 26), et avec une partie de pression de tige (56) qui peut être appliquée axialement contre la tige correctrice (6) et est couplée axialement avec la partie de réglage (52), mais est apte à tourner par rapport à la partie de réglage (52) autour de la direction longitudinale axiale (32), de sorte que des mouvements de rotation de la partie de réglage (52) ne sont pas transmis à la partie de pression de tige (56), et dans lequel les branches de pince (24, 28) entourent en forme de coque la partie de pression de tige (56) et guide celle-ci en déplacement longitudinal et présentent chacune une extrémité distale (46) et une extrémité proximale (48), dans lequel elles s'engagent par leur extrémité distale (46) respective sur la vis à os (4) et peuvent être actionnées à leur extrémité proximale (48) par le chirurgien qui l'utilise, à l'aide d'une pression de doigt, **caractérisé par le fait que** les extrémités distales (46) des deux branches de pince (24, 28) sont conçues de manière à présenter une ouverture de logement (83) ayant une direction transversale radiale (79) pour recevoir la tige correctrice (6) dans cette direction transversale radiale (79) et que cette direction transversale radiale (79) s'étend orthogonalement à l'axe de pivotement (44) des branches de pince (24, 28).

2. Instrument selon la revendication 1, **caractérisé par le fait que** les extrémités distales (46) des deux branches de pince (24, 28) présentent chacune une fente (80, 82) qui s'étend dans la direction longitudinale axiale (32) et est continue dans la direction radiale et qui débouche librement sur la face frontale distale des extrémités distales (46) de sorte que la tige correctrice (6) peut être insérée dans cette fente (80, 82) orthogonalement à son extension longitudinale (72) et dans la direction longitudinale axiale (32) de l'instrument.

3. Instrument selon la revendication 1 ou 2, **caractérisé par le fait que** chaque branche de pince (24, 28) présente deux doigts de pince (76, 78) s'étendant dans la direction longitudinale axiale (32) qui sont espacés les uns des autres dans la direction circonférentielle (74) s'étendant concentriquement à la direction longitudinale axiale (32).

4. Instrument selon la revendication 3, **caractérisé par le fait que**, dans une zone d'extrémité distale (46) et au niveau de leur espacement dans la direction circonférentielle (74), les deux doigts de pince (76, 78) d'une branche de pince (24, 28) présentent des projections (84, 86) qui montrent les unes vers les autres et qui peuvent être appliquées contre différentes branches (8) d'une partie de logement (10) en forme d'U de la vis à os (4) pour la tige correctrice (6) et forment ainsi un dispositif anti-rotation entre la vis à os (4) et l'instrument (2).

5. Instrument selon l'une ou plusieurs des revendications précédentes, **caractérisé par le fait que** les branches de pince (24, 28) présentent, dans une zone d'extrémité distale (46), des nervures (88) s'étendant de manière concentrique radialement à l'intérieur qui peuvent s'engager dans des rainures s'étendant de manière concentrique sur le côté extérieur des branches (8) d'une partie de logement (10) en forme d'U de la vis à os (4) pour former un couplage amovible à engagement positif dans la direction longitudinale axiale (32) entre la vis à os (4) et l'instrument (2).

6. Instrument selon l'une ou plusieurs des revendications précédentes, **caractérisé par le fait que**, lorsque le mouvement d'avance axial de la partie de pression de tige (56) en direction de la vis à os (4) ou bien de la tige correctrice (6) progresse, la partie de pression de tige (56) et au moins une branche de pince (24, 28) forme un couplage à engagement positif de sorte qu'un mouvement d'ouverture de la branche de pince (24, 26) respective par un pivotement autour de l'axe de pivotement (44) est empêché.

7. Instrument selon la revendication 6, **caractérisé par le fait que** la partie de pression de tige (56) s'engage radialement vers l'extérieur entre les doigts de pince (76, 78) d'au moins une branche de pince (24, 28), par une saillie (92) se projetant radialement, et embrasse les doigts de pince (76, 78) lorsque le mouvement d'avance axial de la partie de pression de tige (56) en direction de la vis à os (4) et de la tige correctrice (6) progresse, et empêche ainsi un mouvement d'ouverture de la branche de pince (24, 28) respective par un pivotement autour de l'axe de pivotement (44).

8. Instrument selon la revendication 7, **caractérisé par le fait que**, vue dans un plan de coupe orthogonal à la direction longitudinale axiale (32), ladite saillie (92) se projetant radialement de la partie de pression de tige (56) est réalisée en forme de T ou de champignon.

9. Instrument selon l'une ou plusieurs des revendications précédentes, **caractérisé par le fait que**, dans une articulation de pivotement (30) pour les deux branches de pince (24, 28), au moins une broche d'articulation (42) fait saillie radialement vers l'intérieur et s'engage par son extrémité intérieure dans une rainure de guidage (66) s'étendant dans la direction longitudinale axiale (32) et située sur la face extérieure radiale de la partie de pression de tige (56) et sécurise ainsi celle-ci contre une rotation, mais la guide en déplacement longitudinal dans la direction longitudinale (32).

10. Instrument selon la revendication 9, **caractérisé par le fait que** la rainure de guidage (66) s'étendant dans la direction longitudinale axiale (32), sur la face extérieure radiale de la partie de pression de tige (56), débouche en s'élargissant en V en direction de l'extrémité distale (46) des branches de pince (24, 28).
